# EUROPEAN PATENT APPLICATION

(11) **EP 2 677 315 A1**
(43) Date of publication of application: **25.12.2013**
(21) Application number: 12747135.7
(22) Date of filing: 15.02.2012
(51) Int. Cl.: G01N 33/52

(54) **URINE TEST SHEET**

(30) Priority: 15.02.2011 JP 2011029375
(71) Applicant: Matumura, Takahito, Tokyo 162-0844 (JP)
(72) Inventor: Matumura, Takahito, Tokyo 162-0844 (JP)
(74) Representative: Epping - Hermann - Fischer
(86) International application number: PCT/JP2012/000996
(87) International publication number: WO 2012/111328

(57) **Abstract**

The purpose of the present invention is to provide a urine test sheet which can reduce the workload of a person performing a test through the use of a urine test sheet, caused by the above-mentioned temporary restriction and which makes it possible to obtain a determination result having high reliability. The urine test sheet includes a support, and a reaction reagent which is formed on the support, and by the urine test sheet being immersed in urine to be a specimen and then being taken out, shows a reaction for a prescribed subject to be tested after a prescribed period of time, wherein a detection member in a pad-like shape including the reaction reagent and a reaction-terminating agent having an action causing the reaction to terminate is formed on the support, the reaction-terminating agent is covered with a water-soluble material, and the water-soluble material causes the reaction-terminating agent to act by being dissolved by moisture in the urine.

## Description

### Technical Field

The present invention relates to a urine test sheet that indicates a reaction corresponding to a prescribed subject to be tested, by immersing the sheet in urine to be a specimen and then taking out the same.

### Background Art

In clinical assay, a urine test sheet is widely used by reason that handling is easy, the test is noninvasive, a plurality of subjects to be tested (such as urobilinogen, occult blood, bilirubin, ketone body, glucose, protein, pH, white blood cell and ascorbic acid) can be tested in a short time by one test, etc. In particular, from the above advantage, the urine test is utilized in group examinations for measuring many specimens, and there also is a urine test sheet for home use, which can be utilized even personally.

Many of urine test sheets have, usually, a detection pad composed of a reaction reagent formed on a rectangular sheet. In the measurement with the urine test sheet, a detection pad part of the urine test sheet is immersed in urine to be a specimen and is then taken out, and after a prescribed period of time, from the change in coloring of the detection pad caused by the reaction reagent, determination of various subjects to be tested is performed. The change in coloring of the detection pad can be grasped approximately in a stepwise manner depending on a symptom. Accordingly, there is proposed one in which, while making previously a color tone table for every subject to be tested, a detection pad and the standard color of the color tone table are provided in corresponding positions and shapes for each subject to be tested (for example, see Patent Literature 1). Furthermore, there is proposed such a urine test sheet in which, in order to improve visibility of the change in coloring, a color tone layer (a character or figure) for determination standard which has a color tone being the same as the color tone of unreacted reaction reagent and which does not react with components in urine is provided on the surface of the detection pad and the character in the color tone layer part appears when reaction is performed (for example, see Patent Literature 2).

### Citation List

### Patent Literature

PTL 1: Japanese Utility Model Laid-open 63-99269
PTL 2: Japanese Utility Model Laid-open 06-10866

### Summary of Invention

### Technical Problem

Incidentally, in order to obtain an appropriate determination after taking out a urine test sheet from urine as a specimen, it is necessary to observe the change in coloring after a prescribed period of time determined for every subject to be tested. The prescribed period of time is different depending on a subject to be tested, and is generally such a short period of time as from 30 seconds to 120 seconds. Accordingly, as in group examinations, when the time at which a urine test sheet is taken out of a urine specimen varies, that is, for example, for a subject to be tested for which the above-mentioned prescribed period of time is 30 seconds, when the starting point of measuring 30 seconds differs in accordance with respective urine test sheets, the prescribed period of time has to be measured with time differences of a second unit for respective test sheets, and there is a risk of extremely troublesome work. Furthermore, when there is a plurality of subjects to be tested for which the above-mentioned prescribed period of time is different, the measurement of the measuring time of respective urine test sheets becomes more complicated, and, there were occasionally cases where, substantially, determination was performed before the prescribed period of time or determination was performed considerably after the prescribed period of time. In the case of before the prescribed period of time, the symptom is determined to exhibit a value within the standard value to thereby overlook a symptom, in spite of the symptom actually exhibiting a value exceeding the standard value, and in the case of after the prescribed period of time, the reaction advances too much and useless retest may be forced, although the value is actually within the standard value. That is, in the above-mentioned conventional urine test sheet, there are problems in which an excess workload in terms of time is given to a person performing the test and the reliability of the determination result is damaged.

Furthermore, in urine tests, usually, each of subjects to be tested does not require binary determination of "abnormal" or "normal," but, in many cases, requires at least three-step determination showing the degree of level relative to the prescribed standard value. In the determination, the step is determined on the basis of the change in coloring. However, the change in coloring of the above-mentioned detection pad is continuous but, in contrast, the above-mentioned determination must be performed on the basis of discrete steps, and thus there is a problem in which the determination is difficult. For example, in the case where a large amount of measurements are required to be performed within a given time period such as in group examinations, in conventional technology, it is difficult to read instantly the step of coloring of an individual detection pad, and erroneous determination may have been induced by a person performing the test, in combination with temporal restriction.

Then, in view of the above-mentioned problem, the present invention aims at providing a urine test sheet which can reduce a workload of a person performing a test through the use of a urine test sheet caused by the above-mentioned temporary restriction, and which makes it possible to obtain a determination result having high reliability.

### Solution to Problem

The present invention is a urine test sheet including a support and a reaction reagent which is formed on the support, and by the urine test sheet being immersed in urine to be a specimen and then taken out, shows a reaction corresponding to a prescribed subject to be tested after a prescribed period of time, wherein a detection member in a pad-like shape including the reaction reagent and a reaction-terminating agent having an action causing the reaction to terminate is formed on the support, the reaction-terminating agent is covered with a water-soluble material, and the water-soluble material causes the reaction-terminating agent to act by being dissolved by moisture in the urine.

According to the above-mentioned configuration, after a prescribed period of time, the reaction of the reaction reagent is terminated by the reaction-terminating agent. Therefore, the measurement of individual prescribed time is unnecessary. The amount of the water-soluble material is set so that the reaction-terminating agent is caused to act after prescribed periods of time each specified for each of the subjects to be tested and thus the reaction of the reaction reagent is terminated.

Meanwhile, the shape of the detection member may be one in which the reaction reagent and the reaction-terminating agent covered with the water-soluble material are arranged in a layered form, or may be one contained, in a plural number, with a reaction-terminating agent having the outer surface covered wholly with the water-soluble material and being formed into a granular shape, or the like.

When forming, on the support, detection members corresponding to two or more subjects to be tested having different prescribed periods of time, one having an encircling part around the detection member, in order to block ooze of a reaction-terminating agent contained in the adjacent detection member and shortening of the period of time for terminating the reaction. Furthermore, it may be configured such that the outer surface of the encircling part is contained together with a material that neutralizes the reaction of the reaction-terminating agent.

It may also be possible to set the change in coloring of the detection member according to the reaction of the reaction reagent for the prescribed subject to be tested is set in a stepwise manner by shading, to form, on the support, detection members corresponding to the number of set steps, and to show marks showing the respective steps which are colored in the same color as the coloring of each of the set steps so as to be capable of being read from on respective detection members. In this case, marks colored in the same color as or paler than the coloring of the detection member can not be read from the detection member. Accordingly, among unreadable marks, the step shown by the mark colored in the deepest color is the determination result.

It may also be possible to adopt a configuration such as the followings: the change in coloring caused by the reaction of the reaction reagent for the prescribed subject to be tested is set in a stepwise manner by shading, detection members corresponding to the number of set steps are formed on the support, a mask having window portions capable of observing each of the detection members via films colored, respectively, in the same color as the coloring of each of the set steps is provided, a mark indicating each of the steps corresponding to each of the window portion is written to the mask, and when overlapping the mask upon the support after the reaction, as to the coloring to be observed through the window portions, the coloring can be observed through window portions with the same coloring as the support after the reaction and with a color paler than the coloring. In this case, among those for which the same coloring is observed, the mark showing the highest reaction among steps described for the window portions is the determination result.

Further, it may also be possible that the detection member includes marks indicating each of the steps formed by the reaction reagent, and that a mask having window portions allowing each of the marks to be read via films colored in the same color as the coloring of each of the set steps is overlapped upon the support after the reaction. In this case, from window portions having films colored in the same color as and deeper color than the coloring of the mark after the reaction, the mark is unreadable. Accordingly, the step shown by the palest mark among unreadable marks is the determination result.

Meanwhile, the support may be one provided with window portions having a film allowing each of the marks to be read on the face opposite to the detection member-formed face. That is, according to the configuration, it is possible to determine the measurement result based on the change in coloring of the detection member without using a mask. Furthermore, one in which at least any one of information for identifying a subject, information for specifying the subject to be tested and information showing the determination result corresponding to each of marks is shown on the support by an optically readable code, is acceptable. Furthermore, each of the detection members for every plural and different subject to be tested may be arranged in parallel on the support.

### Advantageous Effects of Invention

The urine test sheet according to the present invention exerts effects such as reducing work loads caused by temporal restriction for a person performing tests and making it possible to obtain determination results with high reliability.

### Brief Description of Drawings

Fig. 1 is a drawing showing a perspective view of the urine test sheet according to the present invention.
Fig. 2 is a drawing showing an enlarged side cross-sectional view of a detection pad part in which a reaction reagent and a reaction-terminating agent are formed in a layered form, in the urine test sheet according to the present invention.
Fig. 3(a) is an enlarged side cross-sectional view of a detection pad part in which the reaction reagent is contained together with the reaction-terminating agent formed into a granular shape, in the urine test sheet according to the present invention, and 3(b) is a drawing showing an enlarged cross-sectional view of the reaction-terminating agent in a granular shape.
Fig. 4 is a drawing showing an enlarged side cross-sectional view of a mode in which an encircling part is provided in the detection pad part of the urine test sheet according to the present invention.
Fig. 5 (a) is a top view of one in which marks showing steps of detection are shown with shading and the detection pad is formed thereon, in the urine test sheet according to the present invention, and 5 (b) is a drawing showing a state of the detection pad after the reaction.
Fig. 6 (a) is a drawing showing a mask having window portions provided with films of different colors, respectively, and having marks shown near the window portions and the urine test sheet according to the present invention, respectively, and 6(b) is a drawing showing a state in which the mask is overlapped upon the above-mentioned urine test sheet.
Fig. 7 (a) is a drawing showing one in which marks are shown using the reaction agent in the specimen pad part of the urine test sheet according to the present invention, and a mask having window portions provided with films of different colors, respectively, and 7(b) is a drawing showing a state in which the above-mentioned mask is overlapped upon the above-mentioned urine test sheet.
Fig. 8 (a) is a drawing showing one in which marks are shown using the reaction agent in the specimen pad part of the urine test sheet according to the present invention, and 8(b) is a drawing showing one in which window portions equipped with films of different colors, respectively, are provided on the backside of the above-mentioned urine test sheet.
Fig. 9 is a drawing showing one that is given a bar cord on the urine test sheet according to the present invention.
Fig. 10 is a drawing showing a mode in which a plurality of specimen pads is arranged in parallel to each other in a single urine test sheet.

### Description of Embodiments

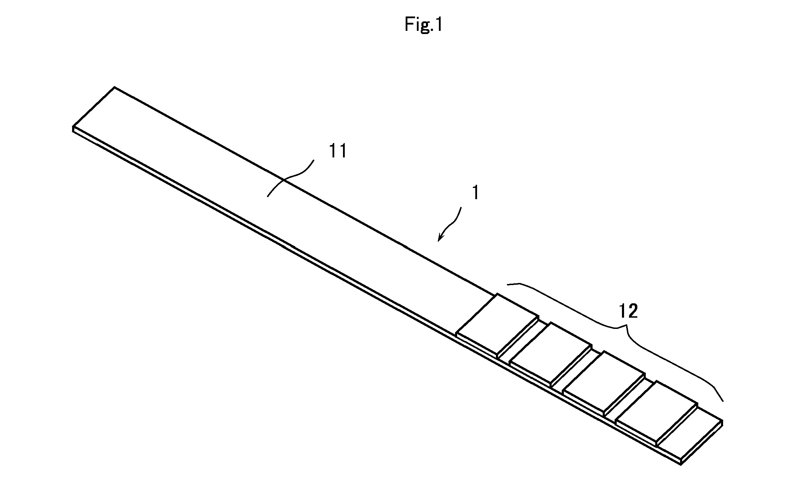

With reference to Fig. 1, 1 is a urine test sheet according to the present invention. In the present embodiment, the shape of the urine test sheet 1 is that, on one end side in a longitudinal direction of a support, that is, a rectangular support sheet 11, four detection members, that is, detection pads 12 are arranged in series, but the embodiment shall not be restricted to the form. In order to test a plurality of subjects to be tested through the use of the single urine test sheet 1, the detection pad 12 is arranged in a plural number, but, in order to measure a single subject to be tested, only one detection pad 12 may be provided. Examples of the above-mentioned subjects to be tested include urobilinogen, occult blood, bilirubin, ketone body, glucose, protein, pH, specific gravity, nitrite, white blood cell, ascorbic acid etc. A urine test is performed, usually, by causing a subject to collect an appropriate amount of urine through the use of a paper cup or the like, and immersing the part of the detection pad 12 of the urine test sheet 1 in the urine specimen in the paper cup and then taking out the same. Accordingly, it becomes necessary to align the detection pad 12 in a range of being immersed in the collected urine, and the above-mentioned form is commonly used. The urine test sheet 1 according to the present embodiment is also, in appearance, basically made to have approximately the same form as the form of conventional urine test sheet.

The material of the support sheet 11 is, although not restricted to a specific one, generally, made of plastic or water-resistant paper. After the urine test sheet being immersed in a urine specimen and then taken out promptly, the detection pad 12 shows, after a prescribed period of time, specific color corresponding to a material or a component amount contained in the urine specimen. This is because a prescribed reaction reagent is contained in the detection pad 12. The principle of the urine test sheet is that, while utilizing the nature of the reaction reagent, the above-mentioned prescribed period of time is set as a required time for the determination in urine tests. The component of the reaction reagent differs according to each of subjects to be tested, and there is performed each of the specifications such as 4-methoxybenzenediazonium tetrafluoroborate for detecting urobilinogen, and tetrabromophenol blue (TBPB) for detecting protein. In the present invention, in addition to the above-mentioned reaction reagent, a reaction-terminating agent for terminating the reaction of these reaction reagents is contained in the detection pad 12. It is configured such that the reaction-terminating agent is covered with a water-soluble material so that the reaction-terminating agent does not act prior to a urine test, and such that, by the immersion of the urine test sheet into a urine specimen, the water-soluble material begins to dissolve by moisture in the urine to cause the reaction-terminating agent to act. The reaction-terminating agent differs corresponding to every reaction reagent, and, for example, may be magnesium chloride, sodium hydroxide, sodium nitrite, phosphate or the like. The amount of the above-mentioned water-soluble material may be set so as to cause the reaction-terminating agent to act after a prescribed period of time specified for each of subjects to be tested and to terminate the reaction of the reaction reagent. The setting can be performed, for example, according to the Fick's law. That is, from the nature that a diffusion flux (flux) of the above-mentioned water-soluble material per unit area and per unit time is proportional to the concentration gradient, the amount of the water-soluble material can be calculated using the period of time until the lapse of the prescribed period of time. Meanwhile, since the concentration distribution of the water-soluble material is considered to vary with the period of time, specifically, the amount of the water-soluble material may be calculated using the Fick's second law.

Incidentally, the prescribed period of time differs depending on each of subjects to be tested. Even in the case of the identical subject to be tested, the prescribed period of time differs depending on the kind, amount or the like of the reaction reagent, and, for example, the period of time differs for every subject to be tested such that around 30 seconds for urobilinogen, around 30 seconds to 60 seconds for occult blood, around 120 seconds for white blood cell, and around 10 seconds for ascorbic acid. The difference in the prescribed periods of time is, as described above, generally a difference in unit of second. Therefore, in the case where a large amount of urine specimens are treated, for example, in group examinations, the above-mentioned prescribed period of time is to be measured for urine test sheets brought in one after another for every subject to be tested, which is a very troublesome work. In addition, when detection pads corresponding to different subjects to be tested (that is, different prescribed periods of time) are formed on a single urine test sheet, furthermore, the measurement of the period of time is extremely difficult, resulting in a state where a precise determination is practically impossible. In the present invention, even when a precise prescribed period of time is not measured for an individual detection pad, the progress of the reaction can be blocked by the above-mentioned reaction-terminating agent after the prescribed period of time. Accordingly, for example, it is sufficient to perform sequentially the determination for one for which the longest period of time (in the case of the above-mentioned example, 120 seconds) among the above-mentioned prescribed periods of time has elapsed. Alternatively, in order to determine whether the reaction is terminated or not, it may also be possible to use a known color tone table to be compared with the detection pad 12 and to perform sequentially the determination from one that shows the same coloring as the color tone table.

Meanwhile, the urine test sheet 1 according to the present invention can be USED by subjects by themselves at home. Conventionally, when a subject uses a test sheet by oneself at home or the like, the subject has to measure on the spot the prescribed period of time specified according to the subject to be tested. In the urine test sheet 1 according to the present invention, however, since the reaction terminates after the prescribed period of time, the measurement of the prescribed period of time becomes unnecessary. Accordingly, the subject can carry the urine test sheet 1 after measurement in health check or send it previously by mail to entrust the determination to an expert, and in addition, a necessary period of time for health check may be shortened. Further, in order to obtain an accurate determination result, subjects has to be in a state of being appropriate to the determination (for example, in a fasting state), but, when the urine test sheet 1 according to the present invention is used, since restriction on eating in accordance with the time of a group examination is unnecessary and the sheet may be used in home at an arbitrary time before eating, a load on subjects can be reduced. Meanwhile, in the present invention, subjects include animals, in addition to mankind.

### Example 1

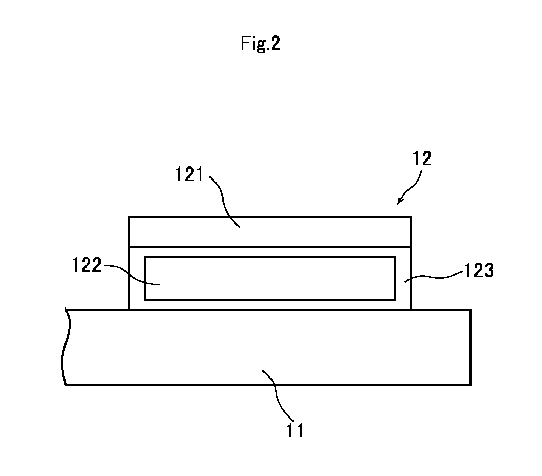

Fig. 2 is a side cross-sectional view showing the enlarged configuration of the detection pad 12 in a first embodiment of the urine test sheet 1 according to the present invention. The detection pad 12 is formed of a reaction reagent 121 and a reaction-terminating agent 122 covered with a water-soluble material 123, which is interposed between the reaction reagent 121 and the support sheet 11. That is, the reaction reagent 121 and the reaction-terminating agent 122 covered with the water-soluble material 123 are arranged in a layered form on the support sheet 11.

### Example 2

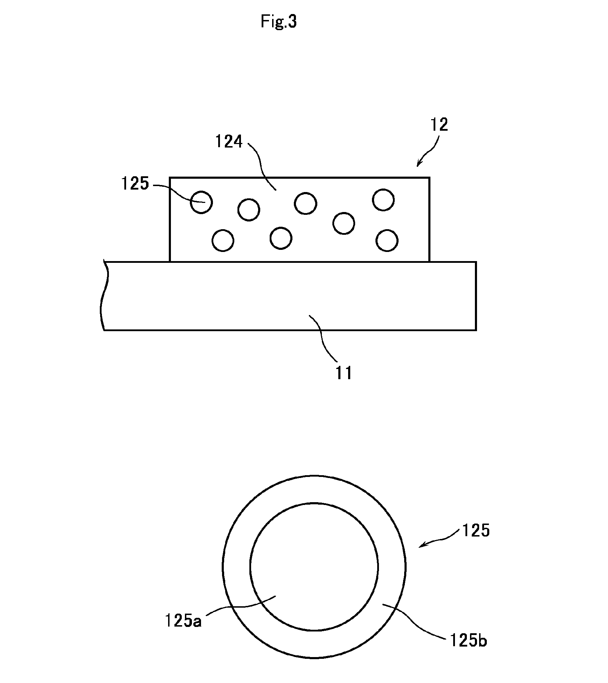

Fig. 3(a) is a side cross-sectional view showing the enlarged configuration of the detection pad 12 in a second embodiment of the urine test sheet 1 according to the present invention. As shown in Fig. 3(b), a reaction-terminating agent 125a is formed in the shape of an approximate spherical body and the whole outer surface thereof is covered with a water-soluble material 125b to form a granular body 125. The detection pad 12 in the present embodiment is formed by containing the granular body 125 on the reaction reagent 124 in a scattered state.

### Example 3

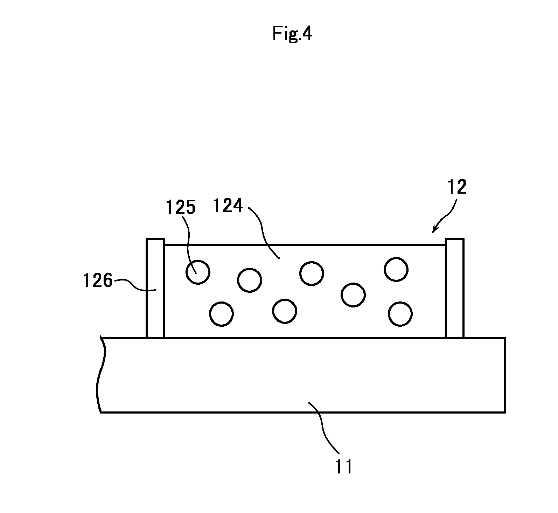

Fig. 4 is a side cross-sectional view showing the enlarged configuration of the detection pad 12 in a third embodiment of the urine test sheet 1 according to the present invention. When detection pads 12 different in the prescribed periods of time are arranged in a plural number in series on the support sheet 11, if a reaction-terminating agent contained in an adjacent detection pad 12 oozes and acts, there may be generated an disadvantage that the amount of the reaction-terminating agent is substantially increased to accelerate the action of terminating the reaction. Therefore, in the present embodiment, in order to block the ooze of the reaction-terminating agent contained in the adjacent detection pad 12, an encircling part 126 is provided around each of the detection pads 12. Meanwhile, in order to block more reliably the ooze of the reaction-terminating agent, a material that neutralizes the reaction of the reaction-terminating agent may be contained in the outer surface of the encircling part 126 (not shown). Meanwhile, in Fig. 4, the detection pad 12 is shown by one containing the granular body 125 described in the second embodiment, but the detection pad to be provided with the encircling part 126 shall not be limited to this, and one obtained by encircling the detection pads 12 described in the first embodiment is also acceptable.

### Example 4

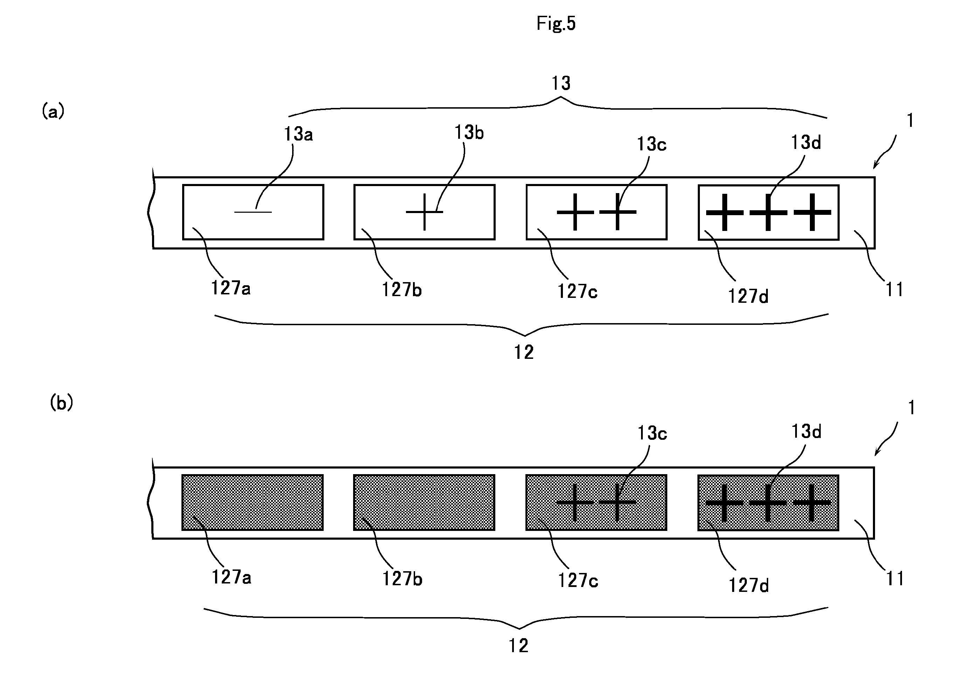

Figs. 5(a) and 5(b) show a urine test sheet which shows the reaction result by a mark for a prescribed subject to be tested. In order to perform the determination from the change in the coloring of the detection pad 12 caused by the reaction of the reaction reagent, in a stepwise manner indices are required depending on the degree of shading of the coloring. So far, there has been present a color tone table that shows determination indices of substantially about 3 to 9 depending on the degree of shading of the coloring for each of subjects to be tested. As in the case of group examinations, however, to determine enormous numbers of urine test sheets while comparing them with the color tone table is a troublesome work and the period of time necessary for the determination per one sheet becomes longer. Even if liberation from the complication of measurement at the prescribed period of time is achieved, when a long period of time is required for the determination treatment, the throughput of the whole test is not enhanced and the temporal load on those performing the test is not dissolved.

Accordingly, the change in the above-mentioned coloring is set in a stepwise manner by the shading, and detection pads 127a to 127d corresponding to the set number of steps are formed on the support sheet 11. In Fig. 5(a), one with 4 steps of from 127a to 127d is shown, but as described above, the number of steps is to be determined in accordance with the subject to be tested. On the support sheet 11, so as to be readable from on each of detection pads 127a to 127d before the reaction, marks 13a to 13d that show each of steps are indicated. In the present embodiment, 4 steps of -, +, ++ and +++ are indicated, but the indication shall not be limited to this if it shows the step. For example, an indication only by numerals such as 1 to 4, an indication showing the number of "+" by a numeral such as +1, +2, etc. are also usable. Each of the marks is colored by the same color as the coloring in each of steps. That is, along with the movement from - to +++, each of the marks is colored so that the density of coloring of the mark also becomes higher.

Fig. 5(b) shows a state where the urine test sheet 1 was immersed in a urine specimen and then taken out, and after that, the prescribed period of time has elapsed. The detection pads 127a to 127d uniformly show a prescribed coloring, in which, as the result of change into this coloring, marks colored in the same color as the coloring and marks colored paler than the coloring are unreadable due to the coloring of the detection pad 12. That is, in Fig. 5(b), marks of 13a(-) and 13b(+) can not be read from on the detection pads 127a and 127b. Among unreadable marks, the step shown by the most deeply colored mark gives the determination result. That is, in Fig. 5(b), such determination result as 13b(+) is drawn. As described above, in the present invention, for the urine test sheet 1 for which the above-mentioned prescribed period of time has elapsed, the determination result can be grasped intuitively, and thus work load on those performing the test can be reduced remarkably. In particular, in the case where a large amount of urine specimens are treated as in the case of group examinations, this effect exerts an extremely large effect.

### Example 5

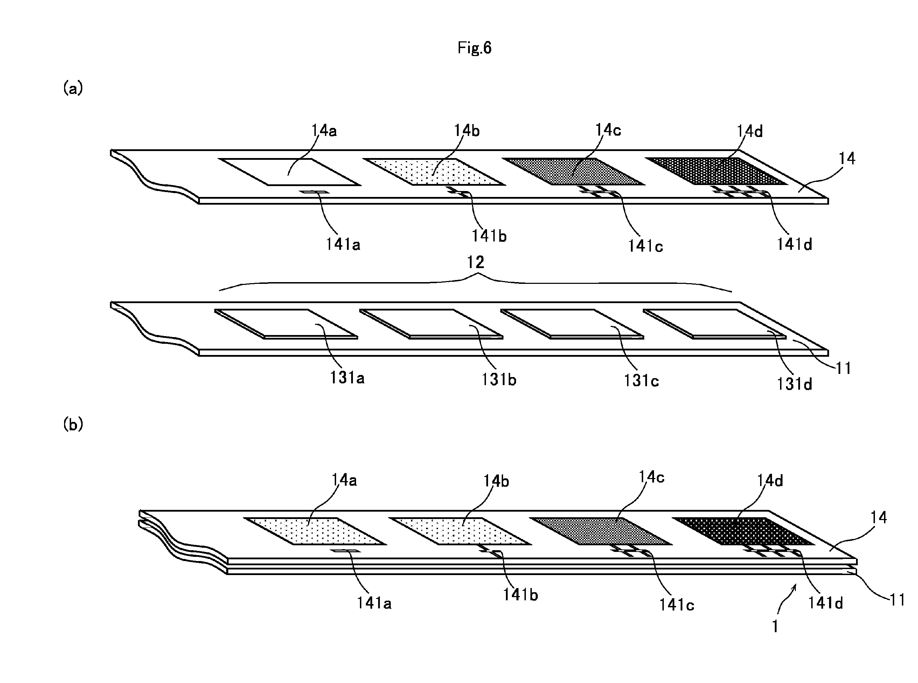

Fig. 6 (a) is a drawing showing a mask 14 for observation use only and the urine test sheet 1 according to the present invention. For detection pads 131a, 131b, 131c and 131d coated on the support sheet 11, no mark showing the step is indicated in particular, in the same way as those shown in Examples 1 to 4, unlike from Example 4 and Examples 6 and 7 to be described later. In the mask 14, window portions 14a, 14b, 14c and 14d are opened so that the detection pads 131a, 131b, 131c and 131d are observable, and to each of windows, while the change in the coloring of the detection pads 131a, 131b, 131c and 131d is set in a stepwise manner by shading, films having the same color as the steps are stuck, respectively. In the vicinity on the lower side of the window portions 14a, 14b, 14c and 14d, marks 141a, 141b, 141c and 141d corresponding to each of the steps are indicated. In the present example, (-) for 141a, (+) for 141b, (++) for 141c, and (+++) for 141d are indicated.

Fig. 6 (b) is a drawing showing a state where the mask 14 is overlapped upon the urine test sheet 1. At this time, the urine test sheet 1 is in a state where the sheet was immersed in a urine specimen and then taken out, and after that, the prescribed period of time has been elapsed. The detection pads 131a to 131d uniformly show prescribed coloring, and as the result of the change into this coloring, from a window portion to which a film of color paler than the coloring among the window portions 14a, 14b, 14c and 14d of the mask 14, observation is performed as color that is the same as the coloring. In the present example, since colors shown by each of the detection pads show the coloring that is the same as the color of the film stuck to the window portion 14b, in the window portion 14a, the observation is performed as the color that is the same as that in the window portion 14b. Accordingly, a mark 141b(+) indicated on the lower side of the window portion 14b is the determination result.

### Example 6

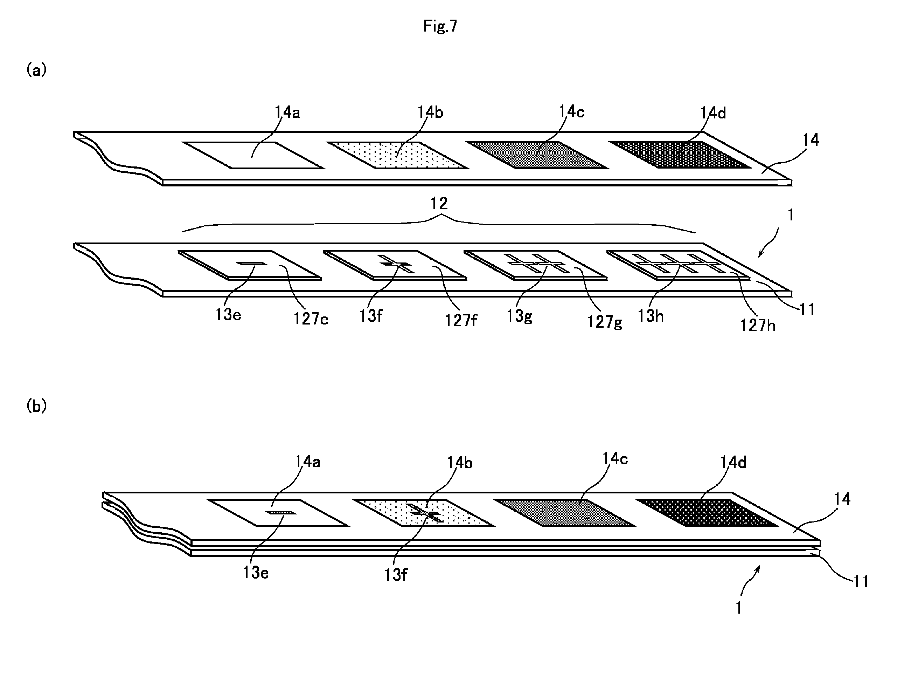

Figs. 7 (a) and 7(b) show a urine test sheet in which the reaction result is shown by a mark for a prescribed subject to be tested, as is the case for the embodiment shown in Figs. 5 (a) and 5(b). Also in Figs. 7(a) and 7(b), as in Figs. 5(a) and 5(b), the change in the above-mentioned coloring is set in a stepwise manner by shading, and detection pads 127e to 127h corresponding to the set number of steps are formed on the support sheet 11. The embodiment in Fig. 7(a) is, however, different from that in Figs. 5 (a) and 5(b) in that the mark is formed by the reaction reagent on the detection pads 127e to 127h. That is, marks 13e(-), 13f(+), 13g(++) and 13h(+++) themselves are indicated by a reaction reagent that changes coloring by a urine specimen. In the present embodiment, in order to read marks 13e to 13h in which the coloring has changed, a mask 14 for exclusive use is employed. In the mask 14, so that each of the marks 13e, 13f, 13g and 13h are readable, window portions 14a, 14b, 14c and 14d are opened, and for each of the window portions, films colored in the same color as the coloring in each of steps are provided, respectively. That is, the reading of each of the marks 13e, 13f, 13g and 13h is performed via films on each of window portions 14a to 14d of the mask 14 after overlapping the mask 14 upon the urine test sheet 1.

Fig. 7(b) shows a state where the urine test sheet 1 was immersed in a urine specimen and then taken out, and after that, the prescribed period of time has been elapsed. The marks 13e to 13h on the detection pads 127e to 127h uniformly show a prescribed coloring, and as the result of the change into this coloring, it is configured such that reading of the mark is impossible through films colored in the same color as or deeper than the coloring. That is, in Fig. 7(b), the marks 13g(+) and 13h(+++) can not be read via films on the window portion 14d of the mask 14. In this case, the step shown by the palest mark among unreadable marks is the determination result for the subject to be tested. That is, in Fig. 5(b), such determination result as 13g(++) is drawn.

### Example 7

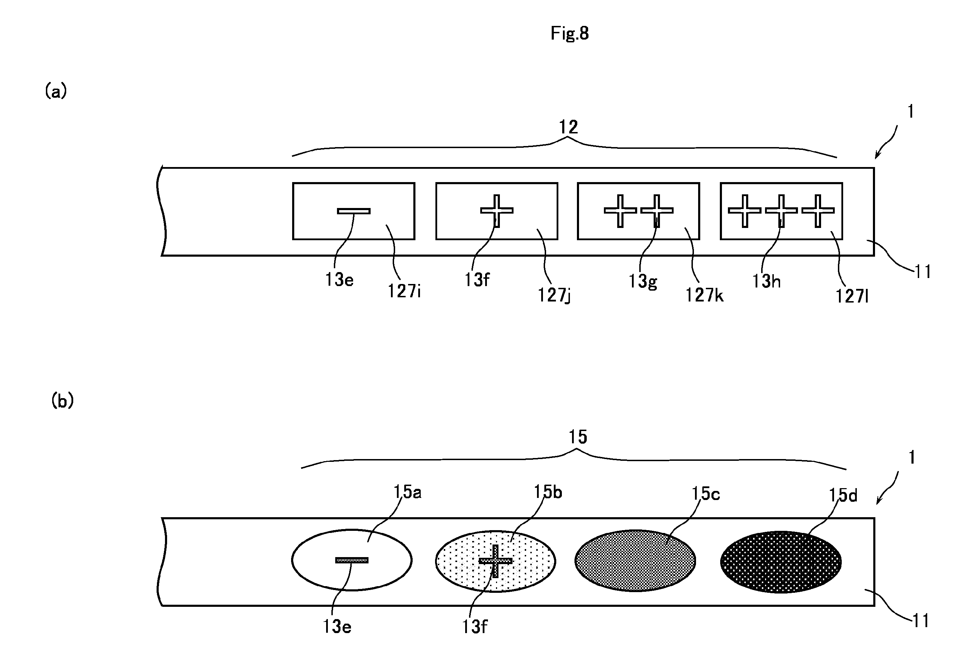

Figs. 8(a) and 8(b) are modified examples of the urine test sheet 1 shown in Figs. 7(a) and 7(b). In the present embodiment, in place of the window portions 14a to 14d of the mask 14 described in Figs. 7(a) and 7(b), a window portion 15 is provided directly on the support sheet 11. That is, as shown in Fig. 8(a), in the face of the support sheet 11 on which the detection pad 12 is formed, in the same way as in Figs. 7(a) and 7(b), marks 13e to 13h are formed by the reaction reagent on each of detection pads 127i to 1271. Each of marks are formed, however, in the same thickness as that of the detection pad 12 (not shown) so that marks can be observed also from the backside of each of the detection pads 127i to 1271.

Fig. 8 (b) shows a state of observing the urine test sheet 1 through the window portion 15, the urine test sheet 1 being in a state where it was immersed in a urine specimen and then taken out, and after that, a prescribed period of time has elapsed. After the immersion in a urine specimen and then the elapse of a prescribed period of time, the marks 13e to 13h formed of the reaction reagent show the change in coloring. At this time, the urine specimen permeates into the reaction reagent and the change in the above-mentioned coloring can be observed also from the backside. Consequently, it is sufficient to open window portions 15a to 15d in positions where the marks 13e to 13h are observable from the face opposite to the face of the support sheet 11 on which the detection pad 12 is formed, and to provide films colored in the same color as the coloring of each of steps for each of the window portions 15a to 15d, respectively. In the present embodiment, as is the case for the fifth embodiment, such a determination result as the mark 13g(++) shown in the position corresponding to the window portion through which the mark is unreadable, that is, the window portion 15c is obtained.

### Example 8

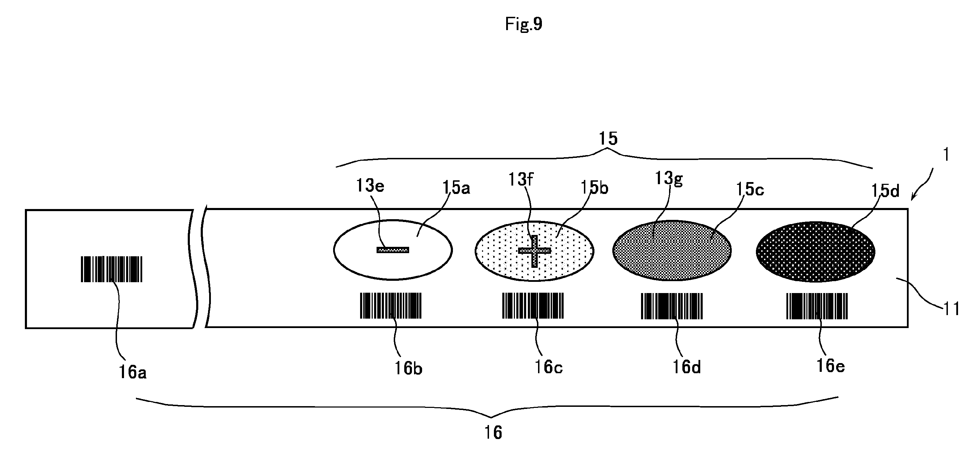

Fig. 9 shows one in which information relating to the urine test sheet 1 is shown on the support sheet 11 by an optically readable code. In urine tests, a work for transcribing determination results to a diagnosis table or the like is generated, and there may be such a risk that, in the case where a large amount of tests are treated in a certain period of time as is the case for group examinations, in addition to the existence of a plurality of determination steps as described above, a transcription error is generated. Consequently, in order to generate no transcription error, a code is indicated so that results can be read with an optical reader. By the indication of the code, for example, it is sufficient that, in the above-mentioned transcription work, each of information is read with a known optical reader and the read information is transferred to an apparatus or the like in which check tables of respective subjects are stored and is transcribed automatically. As to relevant information, at least information for identify a subject, information for specifying a subject to be tested (hereinafter, collectively referred to as "ID information"), and information for showing each of marks shown in the above-mentioned fourth to sixth embodiments may be included. ID information 16a may be indicated on a holding part of the support sheet 11 by which the sheet is held with a hand upon being taken in and out of a paper cup containing a urine specimen, that is, on the end part opposite to the end part in which the detection pad is formed in the longitudinal direction of the support sheet. Information 16b to 16e showing each of marks are shown, in the present embodiment, on the backside of the support sheet 11 in the sixth embodiment, that is, on the lower side of the window portions 15a to 15d while corresponding to each other, and for example, in the fourth and fifth embodiments, the information 16b to 16e may be indicated on the surface of the support sheet 11 (not shown). That is, the information 16b to 16e may be indicated in positions corresponding to the marks after the reaction. In the embodiment, the cord is exemplified by a bar code, but it shall not be restricted to a bar code and, for example, a matrix type two dimensional code etc. are also usable.

### Example 9

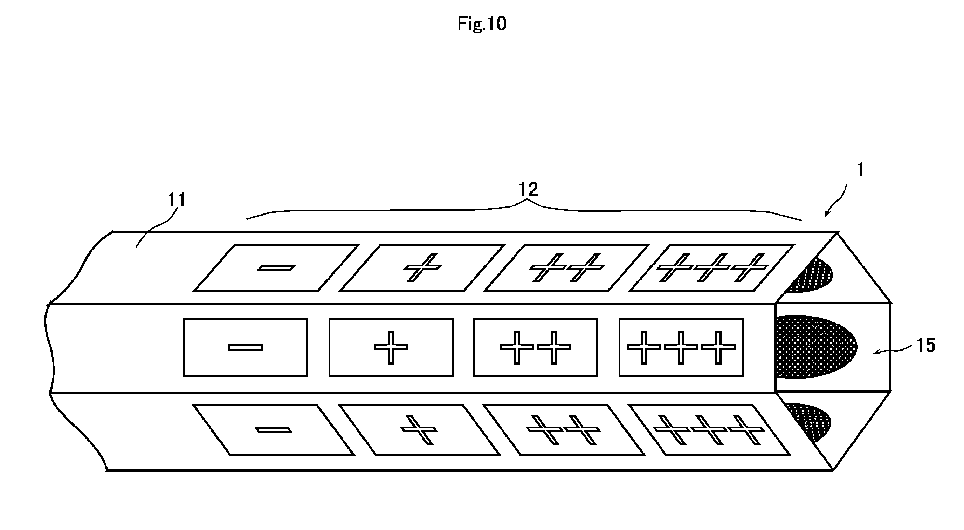

In the fourth embodiment to eighth embodiment, for a single subject to be tested, the detection pads 12 for showing a plurality of steps (4 steps in the present embodiment) are arranged in series. When a plurality of subjects to be tested are to be measured in one test, it is necessary to arrange one, in which the detection pads 12 are arranged in series in number corresponding to the number of steps, in a plurality of rows. In this case, a plurality of urine test sheets 1 may be immersed in a paper cup for collecting a urine specimen, but each becomes individual and, in the case where a large amount of specimens are treated as is the case for group examinations, a sheet may coexist with a sheet of another specimen or may become dispersed and lost. Consequently, as shown in Fig. 10, in order to make measurement possible in a collection amount of a urine specimen that is required for a single subject to be tested, and to make collective measurement possible for every subject, each of the detection pads 12 for every plural and different subject to be tested is arranged in parallel on the support 11. In the present embodiment, the urine test sheet 1 is formed in a hollow polygonal column of an approximately regular hexagon, and, as to the state before measurement, each of the detection pads 12 is arranged in parallel on a flat sheet and, when it is inserted into a paper cup containing a collected urine specimen, so as to make the insertion easy, while setting a support sheet part, in which the detection pad 12 for a single subject to be tested is arranged, to be a fold line, it may be formed into the polygonal column shape. Meanwhile, in the present embodiment, one provided with the window portion on the backside as is described in Figs. 8 (a) and 8 (b) are arranged in parallel, but the embodiment shall not be restricted to this, and the one described in Figs. 5(a), 5(b), 6 (a), 6(b), 7(a), 7(b) or 9 is may also be arranged.

### Reference Signs List

1: urine test sheet
11: support sheet
12: detection pad
13: mark
14: mask
15: window portion
16: code
121, 124: reaction reagent
122, 125a: reaction-terminating agent
123, 125b: water-soluble material

## Claims

1. A urine test sheet comprising:
a support, and
a reaction reagent which is formed on the support, and by the urine test sheet being immersed in urine to be a specimen and then being taken out, shows a reaction for a prescribed subject to be tested after a prescribed period of time, wherein
a detection member in a pad-like shape including the reaction reagent and a reaction-terminating agent having an action causing the reaction to terminate is formed on the support, the reaction-terminating agent is covered with a water-soluble material, and the water-soluble material causes the reaction-terminating agent to act by being dissolved by moisture in the urine.

2. The urine test sheet according to claim 1, wherein
the amount of the water-soluble material is set so as to cause the reaction-terminating agent to act and to terminate a reaction of the reaction reagent after each of the prescribed periods of time specified for each of the subjects to be tested.

3. The urine test sheet according to claim 1 or 2, wherein
the detection member is formed by arranging, in a layered form, the reaction reagent and the reaction-terminating agent covered with the water-soluble material.

4. The urine test sheet according to claim 1 or 2, wherein
the detection member is formed by covering the whole outer surface of the reaction-terminating agent with the water-soluble material to thereby be formed into a granular shape, and by containing, in a plural number, the reaction-terminating agent in the granular shape in the reaction reagent.

5. The urine test sheet according to any one of claims 1 to 4, wherein
detection members corresponding to two or more subjects to be tested which are different in the prescribed period of time are formed on the support, and around each of detection members, an encircling part that blocks an action of the reaction-terminating agent contained in an adjacent detection member is provided.

6. The urine test sheet according to claim 5, wherein
an outer surface of the encircling part includes a material that neutralizes a reaction of the reaction-terminating agent.

7. The urine test sheet according to any one of claims 1 to 6, wherein
change in coloring of the detection member caused by a reaction of the reaction reagent for the prescribed subject to be tested is set in a stepwise manner by shading, detection members corresponding to the number of the set steps are formed on the support, marks showing each of the steps colored in the same color as coloring of each of the set steps are indicated on the support so as to be readable from on each of the detection members, and marks colored in the same color as coloring of a detection member which has changed by the reaction, and colored in color paler than the color are unreadable from on the detection member.

8. The urine test sheet according to any one of claims 1 to 6, wherein
change in coloring caused by a reaction of the reaction reagent for the prescribed subject to be tested is set in a stepwise manner by shading, detection members corresponding to the number of the set steps are formed on the support, a mask having window portions capable of observing each of the detection members is provided via a film colored in the same color as coloring of each of the set steps , a mark indicating each of the steps corresponding to each of the window portions is described on the mask, and when the mask is overlapped upon the support after the reaction, as to coloring observed via the window portion, from window portions having the same coloring as the support after the reaction and having paler color than the coloring, the coloring can be observed.

9. The urine test sheet according to claim 8, wherein
the detection member includes a mark that is formed of the reaction reagent and indicates each of the steps, and when the mask having the window portion is overlapped upon the support after the reaction, the mark is unreadable from window portions having films colored in the same color as coloring of the mark after the reaction and colored in color deeper than the color.

10. The urine test sheet according to claim 9, wherein
the support is provided with a window portion having a film allowing each of the marks to be read, the window portion being provided on the face opposite to the detection member-formed face.

11. The urine test sheet according to any one of claims 7 to 10, wherein
at least one of information identifying a subject, information specifying a subject to be tested, and information showing determination result corresponding to each of the marks is indicated on the support by an optically readable code.

12. The urine test sheet according to any one of claims 7 to 11, wherein
each of the detection members for every plural and different subject to be tested is arranged in parallel on the support.
